# EUROPÄISCHE PATENTANMELDUNG

(11) **EP 4 488 680 A1**
(43) Veröffentlichungstag der Anmeldung: **08.01.2025**
(21) Anmeldenummer: 24175637.8
(22) Anmeldetag: 14.05.2024
(51) Int. Cl.: G01N 33/03

(54) **VERFAHREN ZUR BESTIMMUNG EINES ZUSTANDSPARAMETERS EINES FRITTIERMEDIUMS, VERFAHREN ZUR BESTIMMUNG EINER MENGE IN EIN FRITTIERMEDIUM EINGETRAGENEN WASSERS, SOWIE DETEKTIONSEINRICHTUNG HIERFÜR UND FRITTEUSE**

(30) Priorität: 05.07.2023 DE 102023117755
(71) Anmelder: Testo SE & Co. KGaA, 79822 Titisee-Neustadt (DE)
(72) Erfinder: Zubler, Martin, 79853 Lenzkirch (DE); Landenberger, Benjamin, 79102 Freiburg (DE)
(74) Vertreter: Mertzlufft-Paufler, Cornelius

(57) **Zusammenfassung**

Es wird somit ein Verfahren zur Bestimmung eines Zustandsparameters eines Frittiermediums (3) vorgeschlagen, wobei zur Bestimmung wenigstens ein Ereignisparameter, der wenigstens ein Frittierereignis (10) beschreibt, erfasst und verarbeitet wird. Weiter wird ein Verfahren zur Bestimmung einer Menge in ein Frittiermedium (3) eingetragenen Wassers während eines Frittiervorgangs und/oder Frittierereignisses (10) vorgeschlagen, wobei während des Frittiervorgangs und/oder Frittierereignisses (10) eine Schaumbildung erfasst und ausgewertet wird. Weiter wird eine Detektionseinrichtung (11) zur Detektion von Frittierereignissen (10) einer Fritteuse (12) mit einem vorzugsweise über der Fritteuse (12) angebrachten und/oder auf die Fritteuse (12) gerichteten Sensor (13) und mit einer Recheneinheit vorgeschlagen, wobei die Detektionseinrichtung (11) dazu eingerichtet ist, ein erfindungsgemäßes Verfahren auszuführen. Weiter wird eine Fritteuse (12) vorgeschlagen, welche eine erfindungsgemäße Detektionseinrichtung (11) aufweist (vgl. Figur 3).

## Beschreibung

Die Erfindung betrifft ein Verfahren zur Bestimmung eines Zustandsparameters eines Frittiermediums.

Die Erfindung betrifft weiter ein Verfahren zur Bestimmung einer Menge in ein Frittiermedium eingetragenen Wassers während eines Frittiervorgangs.

Die Erfindung betrifft weiter eine Detektionseinrichtung zur Detektion von Frittierereignissen einer Fritteuse.

Die Erfindung betrifft weiter eine Fritteuse.

Zum Frittieren eines Frittierguts wird dieses in ein üblicherweise mehrfach verwendbares Frittiermedium gegeben. In der Regel handelt es sich hierbei um pflanzliche und/oder tierische Fette und/oder fette Öle. Je nach Zusammensetzung enthält das Frittiermedium gesättigte und/oder ungesättigte Fettsäuren, in der Regel als Triacylglycerole. Während eines Frittiervorgangs unterliegt das Frittiermedium thermischen und/oder chemischen Belastungen, die dessen Qualität mit zunehmendem Frittierfortschritt beeinträchtigen. Der Zustand des Frittiermediums wird zur Sicherung der Qualität und zur Einhaltung gesetzlicher Vorgaben regelmäßig überprüft. Somit können bei einem unzureichenden Zustand des Frittiermediums Gegenmaßnahmen, beispielsweise ein Austausch des Frittiermediums, eingeleitet werden. Diese Überprüfung kann zum einen durch die subjektive Bewertung durch Menschen, zum anderen durch die objektive Messung von qualitätsrelevanten Parametern erfolgen. Beispielsweise können qualitätsrelevante Parameter mittels Messstreifen oder Teststreifen bestimmt werden, wobei diese Art der Bestimmung vergleichsweise teuer und nicht ressourcenschonend sowie aufgrund eines subjektiven Ablesens der Streifen ungenau ist. Bei den qualitätsrelevanten Parametern kann es sich beispielsweise um den Masseanteil polarer Bestandteile (%TPM) oder den Masseanteil freier Fettsäuren (%FFA) handeln. In der Praxis stellt die Messung dieser Parameter einen zeit- und kostenintensiven Vorgang dar, der außerdem geeignetes Personal erfordert.

Die Aufgabe der Erfindung besteht darin, die Ermittlung von qualitätsrelevanten Parametern von Frittiermedien zu vereinfachen.

Zur Lösung der genannten Aufgabe sind erfindungsgemäß die Merkmale des Anspruchs 1 vorgesehen. Insbesondere wird somit zur Lösung der genannten Aufgabe bei Verfahren der eingangs beschriebenen Art erfindungsgemäß vorgeschlagen, dass zur Bestimmung eines Zustandsparameters eines Frittiermediums wenigstens ein Ereignisparameter, der wenigstens ein Frittierereignis beschreibt, erfasst und verarbeitet wird.

Der Ereignisparameter ist hierbei zustandsunabhängig. Dies bedeutet, dass der Ereignisparameter für gleichartige Frittierereignisse übereinstimmen kann, obwohl sich der Zustand des Frittiermediums bei den einzelnen Frittierereignissen unterscheidet. Ein Frittiervorgang kann aus mehreren Frittierereignissen bestehen, wobei ein Frittierereignis von der Hinzugabe eines Frittierguts in ein Frittiermedium bis zur Entfernung des Frittierguts andauert. Der Ereignisparameter kann einen Einfluss des Frittierereignisses auf das Frittiermedium beschreiben. Auf diese Weise kann von dem Ereignisparameter auf einen Zustandsparameter eines Frittiermediums rückgeschlossen werden. Es ist somit nicht erforderlich, den zu bestimmenden Zustandsparameter direkt zu erfassen, beispielsweise zu messen. Somit kann eine Belastung und/oder eine Regeneration des Frittiermediums über den Zustandsparameter bestimmt und/oder verfolgt werden.

Bei einer vorteilhaften Ausgestaltung kann vorgesehen sein, dass erfasste Ereignisparameter akkumuliert werden.

Ereignisparameter können beispielsweise gezählt und/oder miteinander verrechnet werden. Somit kann der Zustandsparameter des Frittiermediums fortlaufend bestimmt werden. Jeweilige Ereignisparameter jeweiliger Frittierereignisse können nacheinander herangezogen werden, um jeweils einen neuen Zustandsparameter des Frittiermediums zu bestimmen. Auf diese Weise kann der Zustand des Frittiermediums über den bestimmten Zustandsparameter verfolgt werden.

Bei einer vorteilhaften Ausgestaltung kann vorgesehen sein, dass wenigstens ein den Zustand des Frittiermediums beschreibender Zustandsparameter in Abhängigkeit von wenigstens einem Startwert und wenigstens einem wenigstens ein Frittierereignis beschreibenden Ereignisparameter berechnet wird.

Auf diese Weise kann ein Startwert herangezogen werden, beispielsweise der Zustandsparameter des Frittiermediums zu einem bestimmten Zeitpunkt, und hiervon ausgehend unter Erfassung und Verarbeitung eines wenigstens ein Frittierereignis beschreibenden Ereignisparameters ein aktualisierter Zustandsparameter bestimmt werden. Somit ist der vorangehende Zustand bereits bekannt und der neue Zustand ergibt sich aus einer Erfassung und Verarbeitung eines Ereignisparameters.

Bei einer vorteilhaften Ausgestaltung kann vorgesehen sein, dass der wenigstens eine Zustandsparameter der Masseanteil polarer Bestandteile an dem Frittiermedium ist und/oder mit diesem korreliert. Alternativ oder zusätzlich kann vorgesehen sein, dass der wenigstens eine Zustandsparameter der Masseanteil freier Fettsäuren an dem Frittiermedium ist und/oder mit diesem korreliert.

Somit können die genannten qualitätsrelevanten Parameter, welche in der Praxis bereits etabliert und Teil von Regeln zur Qualitätssicherung sind, als solche bestimmt werden. Die Messung dieser Parameter ist somit verzichtbar.

Bei einer vorteilhaften Ausgestaltung kann vorgesehen sein, dass wenigstens ein weiterer Zustandsparameter bestimmt und zur Berechnung des Zustandsparameters verwendet wird. Zusätzlich kann vorgesehen sein, dass hierbei wenigstens ein Ereignisparameter für die Berechnung herangezogen wird.

So kann beispielsweise der Masseanteil polarer Bestandteile gemessen und/oder nach einem erfindungsgemäßen Verfahren bestimmt werden und rechnerisch mit wenigstens einem, insbesondere wenigstens zwei, Ereignisparametern kombiniert werden, um einen genaueren Masseanteil freier Fettsäuren zu bestimmen. Dies gilt entsprechend umgekehrt für die Messung des Masseanteils freier Fettsäuren mit nachfolgender Bestimmung des Masseanteils polarer Bestandteile.

Bei einer vorteilhaften Ausgestaltung kann vorgesehen sein, dass der wenigstens eine Ereignisparameter eine Anzahl und/oder eine Dauer und/oder eine Intensität von Frittierereignissen ist und/oder mit wenigstens einer dieser Größen korreliert.

Die Erfindung hat erkannt, dass der Qualitätszustand eines Frittiermediums neben einer etwaigen thermischen Belastung aufgrund des Warmhaltens des Frittiermediums vor allem durch während der Frittierereignisse auftretende thermische und/oder chemische Reaktionen bestimmt wird. Eine Qualitätsverschlechterung eines Frittiermediums ergibt sich vor allem durch den Frittiervorgang selbst. Somit ist es ausreichend, eine Anzahl und/oder eine Dauer und/oder eine Intensität von Frittierereignissen zu bestimmen, um Rückschlüsse auf den Zustandsparameter und somit den Qualitätszustand eines Frittierguts als zu erlauben.

Bei einer vorteilhaften Ausgestaltung kann vorgesehen sein, dass der wenigstens eine Ereignisparameter ein Frittiergut beschreibt und/oder ist und/oder mit diesem korreliert. Zusätzlich kann vorgesehen sein, dass der wenigstens eine Ereignisparameter die Art und/oder Menge und/oder Anzahl des Frittierguts oder der Frittiergüter beschreibt und/oder ist und/oder mit diesen korreliert.

Somit können Eigenschaften des Frittierguts zur Bestimmung des Zustandsparameters herangezogen werden. Beispielsweise ist es möglich, aus erfassten Eigenschaften des Frittierguts, beispielsweise einer Anzahl bestimmter Frittiergüter, auf den Zustandsparameter zu schließen. Die genannten Größen können auch zur Vereinfachung der Bestimmung des Zustandsparameters herangezogen werden.

Bei einer vorteilhaften Ausgestaltung kann vorgesehen sein, dass der wenigstens eine Ereignisparameter eine Menge von Wasser, die in das Frittiermedium eingetragen wurde, ist und/oder mit dieser korreliert.

Insbesondere die Menge eingetragenen Wassers hat einen Einfluss auf den Zustand des Frittierguts und wird somit vorteilhaft zur Bestimmung des Zustandsparameters genutzt. Die Menge von Wasser kann beispielsweise auch aus der Art, Menge und/oder Anzahl von Frittiergütern ermittelt werden.

Bei einer vorteilhaften Ausgestaltung kann vorgesehen sein, dass wenigstens ein Ereignisparameter unter Verwendung wenigstens eines anderen Ereignisparameters berechnet und/oder korrigiert wird.

Beispielsweise kann es sich bei einem Ereignisparameter um die Art von Frittiergut und bei einem anderen Ereignisparameter um die Anzahl des Frittierguts handeln. Hieraus kann beispielsweise eine Menge eingetragenen Wassers berechnet werden, woraus sich der Zustand des Frittiermediums in Form eines Zustandsparameters ergeben kann.

Bei einer vorteilhaften Ausgestaltung kann vorgesehen sein, dass eine Detektion eines Frittierereignisses mittels eines Sensors erfolgt. Hierbei kann vorgesehen sein, dass es sich bei dem Sensor um eine Kamera handelt.

So kann das Frittiermedium mittels einer Kamera überwacht werden und beispielsweise aus einer Blasen- beziehungsweise Schaumbildung ein Frittierereignis abgelesen beziehungsweise erfasst und zur Bestimmung des Zustandsparameters herangezogen werden. So kann beispielsweise die Intensität und/oder Dauer einer Schaumbildung als Ereignisparameter zur Bestimmung eines Zustandsparameters des Frittiermediums herangezogen werden.

Bei einer vorteilhaften Ausgestaltung kann vorgesehen sein, dass der Sensor ein Ultraschallsensor ist. Alternativ oder zusätzlich kann vorgesehen sein, dass der Sensor ein Radarsensor ist. Alternativ oder zusätzlich kann vorgesehen sein, dass der Sensor ein Lidarsensor ist.

Somit können Frittierereignisse, beispielsweise anhand der Schaumbildung in beziehungsweise an dem Frittiermedium auf unterschiedlichste Weise erfasst und zur Bestimmung des Zustandsparameters weiterverwendet werden.

Bei einer vorteilhaften Ausgestaltung kann vorgesehen sein, dass wenigstens ein Ereignisparameter aus einem vorzugsweise digitalen Kassensystem ausgelesen wird. Alternativ oder zusätzlich kann vorgesehen sein, dass wenigstens ein Ereignisparameter von dem Kassensystem berechnet wird.

Somit kann ausgehend von von dem Kassensystem erfassten Bestellungen auf Art, Menge und/oder Anzahl von Frittiergut geschlossen werden, welche als Ereignisparameter in die Bestimmung des Zustandsparameters einfließen können. Eine Erfassung von Frittierereignissen mittels beispielsweise der genannten Sensoren kann somit verzichtbar sein und der Zustandsparameter kann aus den erfassten Frittiergütern berechnet werden.

Bei einer vorteilhaften Ausgestaltung kann vorgesehen sein, dass als wenigstens ein Ereignisparameter ein Einhängen eines Frittierkorbs erfasst wird.

Hierbei kann es sich um das Einhängen des Frittierkorbs in die Fritteuse handeln. Somit kann ermittelt werden, wann ein Frittierereignis beginnt und wann dieses endet. Anzahl und/oder Dauer von Frittierereignissen können somit erfasst und zur Berechnung des Zustandsparameters herangezogen werden.

Alternativ oder zusätzlich sind zur Lösung der genannten Aufgabe erfindungsgemäß die Merkmale des nebengeordneten, auf ein Verfahren zur Bestimmung einer Menge in ein Frittiermedium eingetragenen Wassers während eines Frittiervorgangs und/oder Frittierereignisses gerichteten Anspruchs vorgesehen. Insbesondere wird somit zur Lösung der genannten Aufgabe bei Verfahren der eingangs beschriebenen Art erfindungsgemäß vorgeschlagen, dass während des Frittiervorgangs und/oder Frittierereignisses eine Schaumbildung erfasst und ausgewertet wird.

Somit kann, beispielsweise wie voranstehend beschrieben, die Schaumbildung beziehungsweise Blasenbildung in beziehungsweise an dem Frittiermedium erfasst werden und zur Bestimmung des Zustandsparameters herangezogen werden. Hierin spiegelt sich die Erkenntnis wider, dass der Zustand des Frittiermediums hauptsächlich von Anzahl, Intensität und Dauer von Frittierereignissen und weniger von der Zeit, während der das Frittiermedium bei erhöhten Temperaturen gehalten wird, abhängt.

Alternativ oder zusätzlich sind zur Lösung der genannten Aufgabe erfindungsgemäß die Merkmale des nebengeordneten, auf eine Detektionseinrichtung zur Detektion von Frittierereignissen einer Fritteuse mit einem vorzugweise über der Fritteuse angebrachten und/oder auf die Fritteuse gerichteten Sensor mit einer Recheneinheit gerichteten Anspruchs vorgesehen. Insbesondere wird somit zur Lösung der genannten Aufgabe bei Detektionseinrichtungen der eingangs beschriebenen Art erfindungsgemäß vorgeschlagen, dass die Detektionseinrichtung dazu eingerichtet ist, ein erfindungsgemäßes Verfahren auszuführen.

Somit können die genannten Vorteile erfindungsgemäßer Verfahren bei Detektionseinrichtungen genutzt werden.

Bei einer vorteilhaften Ausgestaltung kann vorgesehen sein, dass der Sensor eine Kamera und/oder Ultraschall- und/oder Radar- und/oder Lidarsensor ist.

Somit können die genannten Sensoren zur Erkennung von Frittierereignissen eingesetzt werden. Die Detektionseinrichtung ist somit zur Ausführung eines erfindungsgemäßen Verfahrens eingerichtet.

Alternativ oder zusätzlich sind zur Lösung der genannten Aufgabe erfindungsgemäß die Merkmale des nebengeordneten, auf eine Fritteuse gerichteten Anspruchs vorgesehen. Insbesondere wird somit zur Lösung der genannten Aufgabe bei Fritteusen der eingangs beschriebenen Art erfindungsgemäß vorgeschlagen, dass diese eine erfindungsgemäße Detektionseinrichtung aufweisen.

Somit können die Vorteile erfindungsgemäßer Detektionseinrichtungen und erfindungsgemäßer Verfahren bei und mit Fritteusen genutzt werden.

Die Erfindung wird nun anhand eines Ausführungsbeispiels näher beschrieben, ist jedoch nicht auf das Ausführungsbeispiel beschränkt. Weitere Ausführungsbeispiele ergeben sich durch Kombination der Merkmale einzelner oder mehrerer Ansprüche untereinander und/oder mit einzelnen oder mehreren Merkmalen des Ausführungsbeispiels.

Es zeigen:
- Figur 1: eine schematische Darstellung der Einflussgrößen auf den Masseanteil freier Fettsäuren eines Frittiermediums als Beispiel für einen qualitätsrelevanten Parameter,
- Figur 2: die Entwicklung des Masseanteils freier Fettsäuren eines Frittiermediums über die Zeit in Abhängigkeit von auftretenden Frittierereignissen,
- Figur 3: eine schematische Darstellung einer erfindungsgemäßen Detektionseinrichtung und Fritteuse.

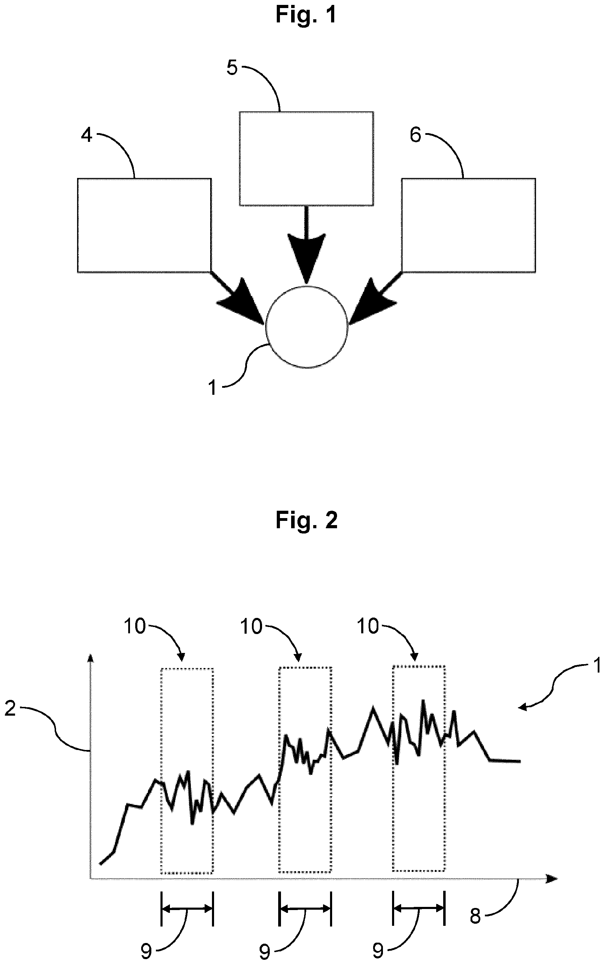

Figur 1 zeigt schematisch, dass eine Erhöhung 1 des Masseanteils freier Fettsäuren 2 in einem Frittiermedium 3 hauptsächlich von drei Einflussgrößen abhängt. Hierbei handelt es sich um Hydrolyse 4, Wanderung 5 und Polymerisation oder Cracken 6 von freien Fettsäuren. Die Hydrolyse 4 von Fettsäureestern erfolgt hierbei aufgrund von Wasser, welches sich in dem Frittiergut 7 befindet, beziehungsweise von diesem in das Frittiermedium 3 eingetragen wird. Ferner kommt es zu einer Erhöhung 1 des Masseanteils freier Fettsäuren 2 durch Wanderung 4 von freien Fettsäuren aus dem Frittiergut 7 in das Frittiermedium 3.

Figur 2 zeigt in einer graphischen Darstellung ein Beispiel für eine Erhöhung 1 des Masseanteils freier Fettsäuren 2 in einem Frittiermedium 3 über die Zeit 8. Es ist erkennbar, dass der Masseanteil freier Fettsäuren 2 in einem Frittiermedium 3 hauptsächlich von Anzahl und Dauer 9 auftretender Frittierereignisse 10 bestimmt wird und über die Zeit 8 ansteigt. Zwischen den Frittierereignissen 10, wobei das Frittiermedium 3 lediglich bei erhöhten Temperaturen gehalten wird, steigt der Masseanteil freier Fettsäuren 2 nicht signifikant an.

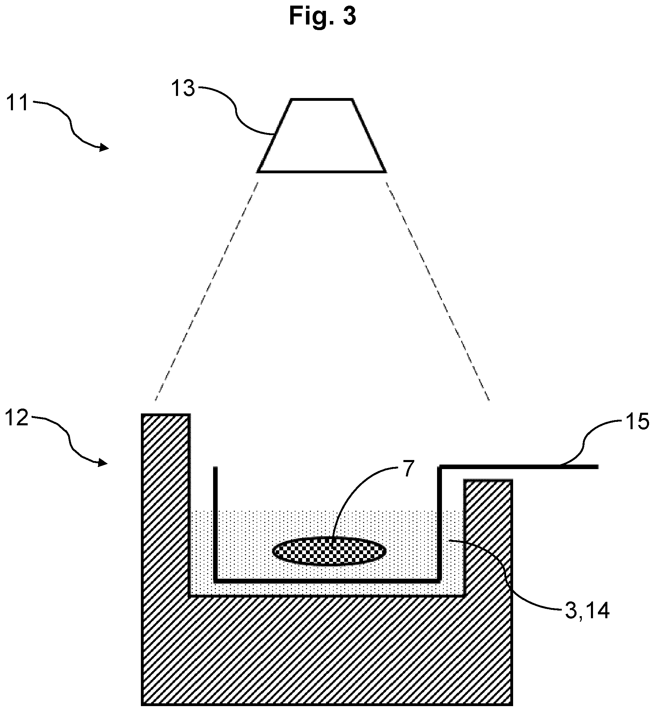

Figur 3 zeigt eine erfindungsgemäße Detektionseinrichtung 11 zur Detektion von Frittierereignissen 10 einer Fritteuse 12, welche hier eine erfindungsgemäße Fritteuse 12 ist, mit einem über der Fritteuse 12 angebrachten und auf die Fritteuse 12 gerichteten Sensor 13 und mit einer nicht näher dargestellten Recheneinheit, wobei die Detektionseinrichtung 11 dazu eingerichtet ist, ein erfindungsgemäßes Verfahren auszuführen.

Der Sensor 13 der Detektionseinrichtung 11 kann hierbei eine Kamera und/oder ein Ultraschall- und/oder Radar- und/oder Lidarsensor sein. Die Fritteuse 12 enthält ein Frittieröl 14 als Beispiel für ein Frittiermedium 3, in welchem sich ein Frittierkorb 15 mit dem Frittiergut 7 befindet. Mittels der Detektionseinrichtung 11 und Fritteuse 12 sind die erfindungsgemäßen Verfahren zur Bestimmung eines Zustandsparameters eines Frittiermediums 3 ausführbar, wobei zur Bestimmung wenigstens ein Ereignisparameter, der wenigstens ein Frittierereignis 10 beschreibt, erfasst und verarbeitet wird.

Hierbei ist es möglich, dass erfasste Ereignisparameter akkumuliert werden. Es kann auch wenigstens ein den Zustand des Frittiermediums 3 beschreibender Zustandsparameter in Abhängigkeit von wenigstens einem Startwert und wenigstens einem wenigstens ein Frittierereignis 10 beschreibenden Ereignisparameter berechnet werden. Der wenigstens eine Zustandsparameter kann hierbei der Masseanteil polarer Bestandteile und/oder der Masseanteil freier Fettsäuren 2 an dem Frittiermedium 3 sein und/oder mit diesen korrelieren. Außerdem kann wenigstens ein weiterer Zustandsparameter bestimmt und zur Berechnung des Zustandsparameters verwendet werden, wobei wenigstens ein Ereignisparameter für die Berechnung herangezogen werden kann.

Der wenigstens eine Ereignisparameter kann eine Anzahl und/oder eine Dauer 9 und/oder eine Intensität von Frittierereignissen 10 sein und/oder mit wenigstens einer dieser Größen korrelieren. Der wenigstens eine Ereignisparameter kann ein Frittiergut 7, insbesondere dessen Art und/oder Menge und/oder Anzahl beschreiben und/oder sein und/oder mit wenigstens einer dieser Grüße korrelieren. Der wenigstens eine Ereignisparameter kann eine Menge von Wasser, die in das Frittiermedium 3 eingetragen wurde, sein und/oder mit dieser korrelieren. Der wenigstens eine Ereignisparameter kann unter Verwendung wenigstens eines anderen Ereignisparameters berechnet und/oder korrigiert werden.

Eine Detektion eines Frittierereignisses 10 kann hierbei mittels eines Sensors 13 und vorzugweise mittels einer Kamera erfolgen. Bei dem Sensor 13 kann es sich auch um einen Ultraschall- und/oder Radar- und/oder Lidarsensor handeln. Der wenigstens eine Ereignisparameter kann auch aus einem vorzugsweise digitalen Kassensystem ausgelesen und/oder von diesem berechnet werden. Als der wenigstens eine Ereignisparameter kann ein Einhängen eines Frittierkorbs 15, insbesondere in die Fritteuse 12, erfasst werden. Ferner ist mit der dargestellten Detektionseinrichtung 11 und der Fritteuse 12 das erfindungsgemäße Verfahren zur Bestimmung einer Menge in ein Frittiermedium 3 eingetragenen Wassers während eines Frittiervorgangs und/oder Frittierereignisses 10, ausführbar, wobei während des Frittiervorgangs und/oder Frittierereignisses 10 eine Schaumbildung erfasst und ausgewertet wird.

Es wird somit ein Verfahren zur Bestimmung eines Zustandsparameters eines Frittiermediums 3 vorgeschlagen, wobei zur Bestimmung wenigstens ein Ereignisparameter, der wenigstens ein Frittierereignis 10 beschreibt, erfasst und verarbeitet wird. Weiter wird ein Verfahren zur Bestimmung einer Menge in ein Frittiermedium 3 eingetragenen Wassers während eines Frittiervorgangs und/oder Frittierereignisses 10 vorgeschlagen, wobei während des Frittiervorgangs und/oder Frittierereignisses 10 eine Schaumbildung erfasst und ausgewertet wird. Weiter wird eine Detektionseinrichtung 11 zur Detektion von Frittierereignissen 10 einer Fritteuse 12 mit einem vorzugsweise über der Fritteuse 12 angebrachten und/oder auf die Fritteuse 12 gerichteten Sensor 13 und mit einer Recheneinheit vorgeschlagen, wobei die Detektionseinrichtung 11 dazu eingerichtet ist, ein erfindungsgemäßes Verfahren auszuführen. Weiter wird eine Fritteuse 12 vorgeschlagen, welche eine erfindungsgemäße Detektionseinrichtung 11 aufweist.

### Bezugszeichenliste

1 Erhöhung
2 Masseanteil freier Fettsäuren
3 Frittiermedium
4 Hydrolyse
5 Wanderung
6 Polymerisation oder Cracken
7 Frittiergut
8 Zeit
9 Dauer
10 Frittierereignis
11 Detektionseinrichtung
12 Fritteuse
13 Sensor
14 Frittieröl
15 Frittierkorb

## Patentansprüche

1. **Verfahren** zur Bestimmung eines Zustandsparameters eines Frittiermediums (3), wobei zur Bestimmung wenigstens ein Ereignisparameter, der wenigstens ein Frittierereignis (10) beschreibt, erfasst und verarbeitet wird.

2. Verfahren nach dem vorangehenden Anspruch, wobei erfasste Ereignisparameter akkumuliert werden.

3. Verfahren nach einem der vorangehenden Ansprüche, wobei wenigstens ein den Zustand des Frittiermediums (3) beschreibender Zustandsparameter in Abhängigkeit von wenigstens einem Startwert und wenigstens einem wenigstens ein Frittierereignis (10) beschreibenden Ereignisparameter berechnet wird.

4. Verfahren nach dem vorangehenden Anspruch, wobei der wenigstens eine Zustandsparameter der Masseanteil polarer Bestandteile und/oder der Masseanteil freier Fettsäuren (2) an dem Frittiermedium (3) ist und/oder mit diesem oder diesen korreliert/korrelieren.

5. Verfahren nach einem der vorangehenden Ansprüche, wobei wenigstens ein weiterer Zustandsparameter bestimmt und zur Berechnung des Zustandsparameters verwendet wird, insbesondere wobei wenigstens ein Ereignisparameter für die Berechnung herangezogen wird.

6. Verfahren nach einem der vorangehenden Ansprüche, wobei der wenigstens eine Ereignisparameter eine Anzahl und/oder eine Dauer (9) und/oder eine Intensität von Frittierereignissen (10) ist und/oder mit wenigstens einer dieser Größen korreliert.

7. Verfahren nach einem der vorangehenden Ansprüche, wobei der wenigstens eine Ereignisparameter ein Frittiergut (7), insbesondere dessen Art und/oder Menge und/oder Anzahl, beschreibt und/oder ist und/oder mit wenigstens einer dieser Größen korreliert.

8. Verfahren nach einem der vorangehenden Ansprüche, wobei der wenigstens eine Ereignisparameter eine Menge von Wasser, die in das Frittiermedium (3) eingetragen wurde, ist und/oder mit dieser korreliert.

9. Verfahren nach einem der vorangehenden Ansprüche, **dadurch gekennzeichnet, dass** wenigstens ein Ereignisparameter unter Verwendung wenigstens eines anderen Ereignisparameters berechnet und/oder korrigiert wird.

10. Verfahren nach einem der vorangehenden Ansprüche, wobei eine Detektion eines Frittierereignisses (10) mittels eines Sensors (13), vorzugsweise mittels einer Kamera erfolgt.

11. Verfahren nach einem der vorangehenden Ansprüche, wobei der Sensor (13) ein Ultraschall- und/oder Radar- und/oder Lidarsensor ist.

12. Verfahren nach einem der vorangehenden Ansprüche, wobei wenigstens ein Ereignisparameter aus einem vorzugsweise digitalen Kassensystem ausgelesen und/oder von diesem berechnet wird.

13. Verfahren nach einem der vorangehenden Ansprüche, wobei als wenigstens ein Ereignisparameter ein Einhängen eines Frittierkorbs (15) erfasst wird.

14. **Verfahren** zur Bestimmung einer Menge in ein Frittiermedium (3) eingetragenen Wassers während eines Frittiervorgangs und/oder Frittierereignisses (10), wobei während des Frittiervorgangs und/oder Frittierereignisses (10) eine Schaumbildung erfasst und ausgewertet wird.

15. **Detektionseinrichtung** (11) zur Detektion von Frittierereignissen (10) einer Fritteuse (12) mit einem vorzugsweise über der Fritteuse (12) angebrachten und/oder auf die Fritteuse (12) gerichteten Sensor (13) und mit einer Recheneinheit, wobei die Detektionseinrichtung (11) dazu eingerichtet ist, ein Verfahren gemäß einem der Ansprüche 1 bis 14 auszuführen.

16. Detektionseinrichtung (11) nach dem vorangehenden Anspruch, **dadurch gekennzeichnet, dass** der Sensor (13) eine Kamera und/oder ein Ultraschall- und/oder Radar- und/oder Lidarsensor ist.

17. **Fritteuse** (12) mit einer Detektionseinrichtung (11) nach einem der Ansprüche 15 oder 16.
